# EUROPEAN PATENT SPECIFICATION

(11) **EP 4 135 794 B1**
(45) Date of publication and mention of the grant of the patent: **06.08.2025**
(21) Application number: 21720959.2
(22) Date of filing: 12.04.2021
(51) Int. Cl.: A61M 5/20, A61M 5/315, A61M 5/32

(54) **INJECTION DEVICE WITH DISENGAGEMENT FEATURE AND METHOD FOR DISENGAGING A PLUNGER FROM A POWER SOURCE**
EINSPRITZVORRICHTUNG MIT ENTKUPPLUNGSFUNKTION UND VERFAHREN ZUM ENTKUPPELN EINES KOLBENS VON EINER ENERGIEQUELLE
DISPOSITIF D'INJECTION AVEC FONCTION DE DÉSACCOUPLEMENT ET PROCÉDÉ DE DÉSACCOUPLEMENT D'UN PISTON À PARTIR D'UNE SOURCE DE PUISSANCE

(30) Priority: 14.04.2020 US 202063009900 P
(43) Date of publication of application: 22.02.2023
(73) Proprietor: West Pharmaceutical Services, Inc., Exton, PA 19341 (US)
(72) Inventor: LAURENCE, Lawton, Edward, Chester Springs, PA 19425 (US); NUTT, Lauren, Exton, PA 19341 (US)
(74) Representative: Durling, Barbara Eleanor Ravenhill
(86) International application number: PCT/US2021/026814
(87) International publication number: WO 2021/211417

(56) References cited:
- EP-A1- 2 812 051
- EP-A1- 3 366 336
- EP-B1- 2 812 051
- WO-A1-03/097133

## Description

### TECHNICAL FIELD

This application is directed to an injection device. Specifically, this application relates to an injection device with a translating disengagement feature for disengaging a plunger from a power pack.

### BACKGROUND

Handheld injection devices for the injection of a medicament into a patient can be powered through many different mechanisms, including springs, electric motors, etc. Such injection devices can be utilized to insert a needle of the injection device into a patient and/or actuate a plunger through a cartridge attached to the needle so as to inject the medicament into the patient. The power sources utilized in such injection devices can be relatively powerful, and thus it can be desirable to control the amount of force applied by the power source to the cartridge and/or plunger. However, design interests and other limitations can impose significant constraints on any mechanism utilized to limit force transfer. Such design interests and limitations can include a desire to minimize the size of the overall injection device, requisite device complexity, and the use of materials inefficient for bulk manufacturing.

As such, there is a need for a handheld injection device that includes a system for limiting force transfer from a power source.

WO03097133A1 discloses an injection device with a needle which, when the device is operated, is first caused to project, then liquid is forced out through it, and finally the needle is automatically retracted. The needle extends forwardly from a capsule that can slide longitudinally within a barrel-like body, a relatively weak spring normally maintaining the capsule and needle retracted. A more powerful spring acts oppositely on a plunger formed by rod parts which, when released, shoots the capsule forward by acting on the liquid therein, and then forces the liquid out through the projecting needle. At the end of the forward stroke the plunger and capsule are decoupled and the weak spring returns the exhausted capsule and its needle to the retracted position. A lost motion connection provided by a piston of the rod part acts as a damper in a cylinder of the rod part, to ensure that the full dose is ejected from the needle before decoupling occurs.

EP2812051A1 discloses a low retraction activation force plunger sub-assembly for an automatic injector which includes: a plunger outer having one or more engagement prongs, a plunger inner having a shoulder, and a plunger biasing member retained in a first energized state between said plunger outer and plunger inner when the engagement prongs of the plunger outer are releasably engaged with the shoulder of the plunger inner. An automatic injector includes a housing, an activation mechanism, an actuation mechanism, and a syringe cartridge having the plunger sub-assembly and a needle assembly, wherein the actuation mechanism comprises an actuation biasing member residing in an initial energized state substantially within an upper portion of an actuation pill. A method of assembling the automatic injector includes the steps of: assembling the plunger sub-assembly and inserting the plunger sub-assembly into the housing such that a proximal end of the plunger sub-assembly contacts the actuation pill.

EP3366336A1 discloses an auto-injector for administering a medicament and a method for operating it, the auto-injector comprising: a tubular chassis and a carrier subassembly, comprising a tubular carrier slidably arranged in the chassis. The carrier contains a syringe, a drive spring and a plunger for forwarding load of the drive spring to a stopper arranged in the syringe. The syringe is locked for joint axial translation with the carrier. A control spring is connectable to the carrier by first interlock means for needle insertion, wherein the whole carrier subassembly is advanced. Second interlock means are arranged for releasing the drive spring when the carrier has at least almost reached an injection depth thus delivering the medicament. The first interlock means are arranged for decoupling the control spring from the carrier and coupling it to the chassis for advancing it over the needle into a needle safe position.

### SUMMARY

The invention lies in the injection device of claim 1. Further embodiments are given in the dependent claims.

A further aspect of the present disclosure outside the subject-matter of the claims is a method of disconnecting a power source of an injection device from a plunger actuation assembly, where the plunger actuation assembly is configured to drive a plunger disposed within a chamber of a cartridge, the chamber being configured to hold a medicament. The method includes actuating the power source to drive the plunger actuation assembly a first distance, where a second disengagement element is configured to couple the plunger actuation assembly to the power source, and translationally decoupling a first disengagement element from the plunger actuation assembly. The method also includes driving the plunger actuation assembly a second distance and translationally decoupling, via the first disengagement element, the second disengagement element and the power source from the plunger actuation assembly.

### BRIEF DESCRIPTION OF THE DRAWINGS

The foregoing summary, as well as the following detailed description, will be better understood when read in conjunction with the appended drawings. The drawings show illustrative embodiments of the disclosure. It should be understood, however, that the application is not limited to the precise arrangements and instrumentalities shown.
FIG. 1 is a perspective view of an injection device according to an embodiment of the present disclosure;
FIG. 2 is a cross-sectional view of the injection device of claim 1 with the cover removed and the injection device in an initial, unactuated configuration, taken along line 2-2 shown in FIG. 1;
FIG. 3 is an exploded view of certain components of the injection device shown in FIG. 1;
FIG. 4 is a perspective view of a first disengagement element of the injection device shown in FIG. 1;
FIG. 5A is a perspective view of a second disengagement element of the injection device shown in FIG. 1;
FIG. 5B is an enhanced portion of the cross-sectional view shown in FIG. 2;
FIG. 6 is a cross-sectional view of the injection device of claim 1 with the cover removed and the injection device in an injection configuration prior to medicament injection, taken along line 2-2 shown in FIG. 1;
FIG. 7 is a cross-sectional view of the injection device of claim 1 with the cover removed and the injection device in an injection configuration after medicament injection, taken along line 2-2 shown in FIG. 1;
FIG. 8 is a cross-sectional view of the injection device of claim 1 with the cover removed and the injection device in a final retracted configuration after medicament injection, taken along line 2-2 shown in FIG. 1; and
FIG. 9 is a process flow diagram of a method of disconnecting a power source of an injection device from a plunger actuation assembly.

### DETAILED DESCRIPTION OF ILLUSTRATIVE EMBODIMENTS

Described herein is an injection device 10 powered by a power source 145 for injecting a medicament into a patient. Certain terminology is used to describe the injection device 10 in the following description for convenience only and is not limiting. The words "right," "left," "lower," and "upper" designate directions in the drawings to which reference is made. The words "inner" and "outer" refer to directions toward and away from, respectively, the geometric center of the description to describe the injection device 10 and related parts thereof. The words "forward" and "rearward" refer to directions in a longitudinal direction L and a direction opposite the longitudinal direction L along the injection device 10 and related parts thereof. The terminology includes the above-listed words, derivatives thereof and words of similar import. Unless otherwise specified herein, the term "longitudinal" is used to describe a longitudinal directional component of various components of the injection system 10 as designated by the longitudinal direction L, while the term "radial" is used to describe a radial directional component of the injection system 10 that is perpendicular to the longitudinal direction L as designated by the radial direction R. The radial direction R includes the radial direction R as shown in each figure, as well as any other direction that is perpendicular to the longitudinal direction L.

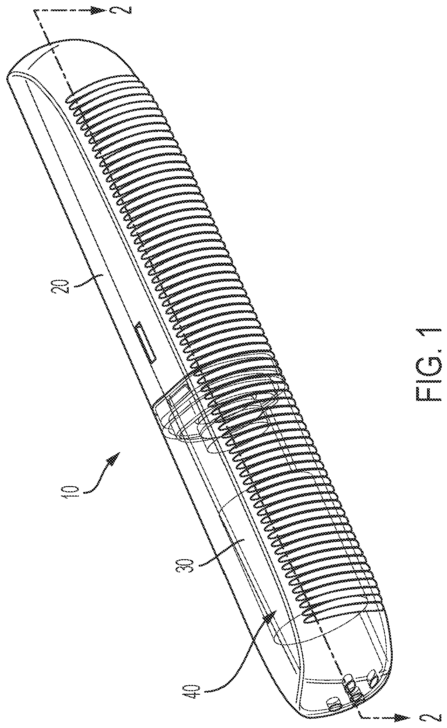

FIGS. 1-2 depict an injection device 10 according to an embodiment of the current disclosure. The injection device 10 is configured to be manually held and actuated by a patient. The injection device 10 can include a substantially hollow housing 20, which can be comprised of molded plastic or metal, for example. The housing 20 can be substantially elongate along the longitudinal direction L, and can define a cavity 24 therein, where components of the injection device 10 are configured to be supported by the housing 20 within the cavity 24. A portion of the housing 20 can be transparent to allow patient monitoring of the contents of a cartridge 50 contained within the housing 20, where the cartridge 50 will be described further below. A cap 30 can be releasably attached to the housing 20, where the cap 30 is configured to shield certain components of the injection device 10 to maintain sterility of certain components and/or protect a patient from unintended needle contact. The cap 30 can be molded from plastic, though other materials are contemplated. Additionally, as depicted the cap 30 can be substantially transparent. However, it is also contemplated that the cap 30 can be opaque and comprise a variety of colors and/or markings as desired.

The housing 20 is configured to at least partially contain a cartridge 50, where the cartridge 50 is prefilled with a medicament to be injected into a patient. The depicted injection device 10 can be supplied to an end patient with a cartridge 50 preinstalled within the housing 20, though it is contemplated that in other embodiments the cartridge 50 can be removable from within the housing 20 and replaced. The cartridge 50 has a body 52 that extends from a first end 50a to a second end 50b opposite the first end 50a along the longitudinal direction L. The cartridge 50 further defines a chamber 54 therein, where the chamber 54 extends from a proximal opening 56 defined at the first end 52a of the body 52 to a distal opening 58 defined at the second end 52b of the body 52. The injection device 10 can further include a needle 60 extending from the cartridge 50. The injection device 10 can further include a elastomeric tip cap (not shown) that can be disposed around the needle 60 so as to create a sterile seal on the needle 60, as well as a rigid needle shield (not shown) that can be utilized to hold the tip cap in place and allow for removal of the tip cap and rigid needle shield. A coupler 59 can be attached to the second end 52b of the body 52, where the coupler 59 is configured to secure the needle 60 to the cartridge 50. Though a coupler 59 is specifically shown as attaching the needle 60 to the cartridge 50, it is contemplated that the cartridge 50 can be alternatively configured without the coupler 59. For example, in such embodiments the needle 60 can be directly attached to the cartridge 50. When attached the cartridge 50, the needle 60 can be in fluid communication with the distal opening 58, and thus the chamber 54, such that medicament can flow through the needle 60 from the cartridge 50 and into a patient upon actuation of the injection device 10. In addition to the cap 30, the injection device 10 can include a needle shield 40 attached to the housing 20, where the needle shield 40 is configured to selectively shield the patient from the needle 60. The injection device 10 can further include a return spring 160 disposed at least partially within the needle shield 40, where opposing ends of the return spring 160 are configured to engage the needle shield 40 and cartridge 50, respectively. The function of the return spring 160 will be described in greater detail below.

Continuing with FIGS. 1-3, the injection device 10 includes a power pack housing 65 disposed within the cavity 24 of the housing 20 and attached to the housing 20. In one embodiment, the power pack housing 65 is a separate component from but integral with the housing 20, though it is contemplated that the power pack housing 65 and the housing 20 could be monolithic. The power pack housing 65 can have a body 67 that extends from a first end 67a to a second end 67b opposite the first end 67a along the longitudinal direction L. The power pack housing 65 can also include an outer surface 67c and an inner surface 67d opposite the outer surface 67c, where the inner surface 67d defines a channel 68 extending through the body 67 from the first end 67a to the second end 67b. The channel 68 can have a generally circular cross-section and can define varying diameters across its length, i.e., can be tiered along the longitudinal direction L. In one instance, the inner surface 67d can define a ledge 69 facing the channel 68, where the function of the ledge 69 will be described further below. The ledge 69 can define a stepped region of the inner surface 67d where two portions of the channel 68 having differing diameters meet. The ledge 69 can extend substantially circumferentially about the entirety of the inner surface 67d, though it is contemplated that the ledge 69 may only extend partially about the inner surface 67d.

The injection device 10 can further include a proximal housing 70 disposed within the cavity 24 of the housing 20 and attached to the housing 20. The proximal housing 70 can define a chamber 74 therein, where the chamber 74 is configured to receive certain components of the injection device 10, as will be described below. Specifically, the injection device 10 includes a power source 145 configured to be at least partially received within the chamber 74 of the proximal housing 70 and configured to drive the plunger actuation assembly 100 such that the plunger 80 forces the medicament from the chamber 74, as will be described further below. In the depicted embodiment, the power source 145 comprises a power pack spring 150. However, it is contemplated that the power source 145 can be differently configured. For example, it is contemplated that the power source 145 can alternatively comprise an electromechanical motor, pneumatic actuator, etc.

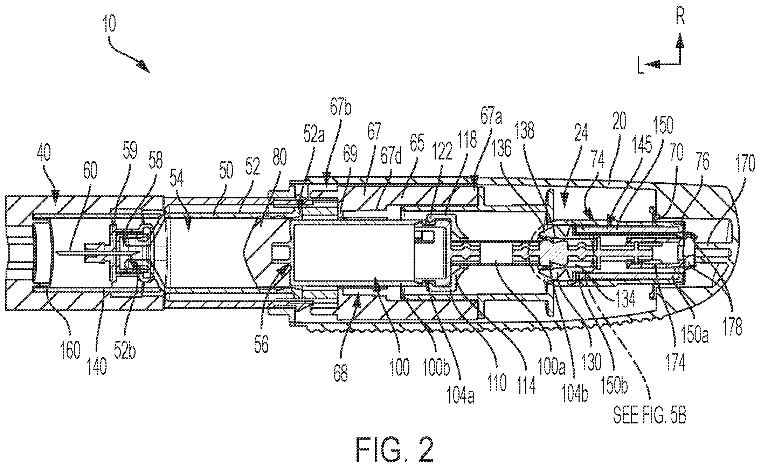

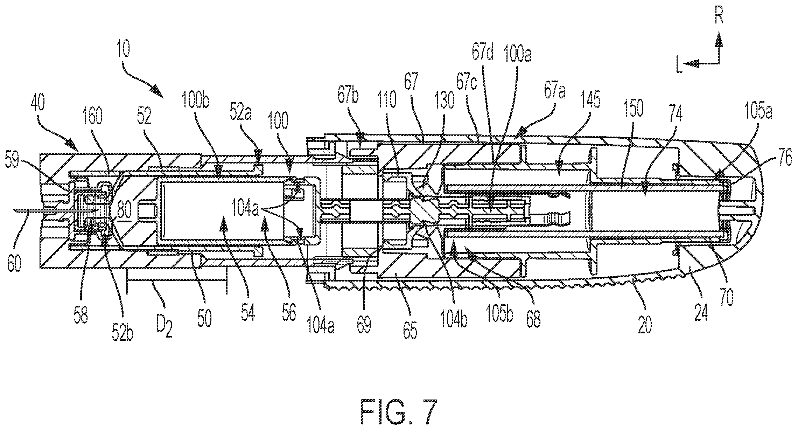

The power pack spring 150 extends from a first end 150a to a second end 150b opposite the first end 150a along the longitudinal direction L. The power pack spring 150 can comprise a metal coiled spring, and can be configured to expand from a first length (such as shown in FIG. 2) to a second length (such as shown in FIG. 7) so as to operate certain aspects of the injection device 10, as will be described further below. In the depicted embodiment, the power pack spring 150 is a single spring. However, it is contemplated that in other embodiments the power pack spring 150 can comprise a set or series of springs. The first end 150a of the power pack spring 150 can engage a ledge 76 defined by the proximal housing 70, while the second end 150b of the power pack spring 150 can engage a portion of a second disengagement element 130, which will be described further below. The exact embodiment of power pack spring 150 utilized in the injection device 10 can be a design choice to account for the specific force and/or spatial requirements of a particular injection device.

The injection device 10 also includes a plunger 80 disposed within the chamber 54 of the cartridge 50, where the plunger 80 is configured to selectively force the medicament from the chamber 54. The plunger 80 can comprise a conventional rubber or elastomeric plunger, where the plunger is configured to form a fluid seal with the inner surface of the cartridge 50 to prevent the medicament from flowing past the plunger 80 and escaping the cartridge 50. Though the plunger 80 creates a fluid seal with the cartridge 50, the plunger 80 can be translatable through the chamber 54 of the cartridge 50 along the longitudinal direction L. To accomplish this, the injection device 10 includes a plunger actuation assembly 100 is configured to drive the plunger 80. In the depicted embodiment, the plunger actuation assembly 100 can be comprised of a plurality of interconnected rods having varying diameters. However, it is contemplated that in other embodiments, the plunger actuation assembly can be comprised of a single, integrally formed body. The plunger actuation assembly 100 can include a first portion 100a and a second portion 100b connected to the first portion 100a and extending from the first portion 100a along the longitudinal direction L. The second portion 100b can define a diameter that is larger than the diameter of the first portion 100a. The diameter of each of the first and second portions 100a, 100b can be the same or differ along their lengths as desired. Though a plunger actuation assembly 100 including two portions is explicitly disclosed, it is contemplated that in other embodiments the plunger action assembly can be comprised of more or less than two portions, such as one portion, three portions, four portions, etc. Initially, the plunger actuation assembly 100 can be spaced from the plunger 80, and can engage the plunger 80 only after some initial displacement caused by the power source 145. However, it is also contemplated that the plunger actuation assembly 100 can be initially attached to the plunger 80.

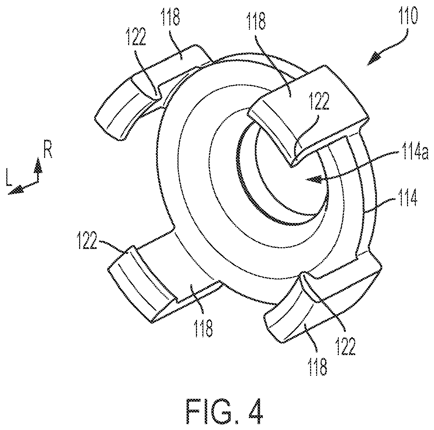

Referring to FIG. 4, the injection device 10 includes a first disengagement element 110. The first disengagement element 110 can define a body 114 that can be substantially disc-shaped, such that the body 114 defines a channel 114a extending therethrough. The channel 114a can be specifically sized such that the channel 114a is configured to receive a portion of the plunger actuation assembly 100. Specifically, the channel 114a can be configured to receive the first portion 100a of the plunger actuation assembly 100, such that the first disengagement element 110 is concentrically disposed around the first portion 100a of the plunger actuation assembly 100. The first disengagement element 110 can also define at least one tab 118 extending longitudinally from the body 114, and each tab 118 can include a protrusion 122 extending inwards therefrom along the radial direction R. As a result, each tab 118 and protrusion 122 combination can collectively substantially define a hook-like shape. In the depicted embodiment, the first disengagement element 110 can include four tabs 118 circumferentially spaced apart, and likewise four corresponding protrusions 122. However, in other embodiments, it is contemplated that the first disengagement element 110 can include different numbers of tabs 118 and protrusions 122, such as one, two, three, five, six, seven, etc. As depicted, the tabs 118 are equidistantly circumferentially spaced apart. In other embodiments, the tabs 118 can be non-equidistantly spaced apart circumferentially. Each component of the first disengagement element 110 can be comprised of a substantially flexible material, such as plastic, though other materials are contemplated, such that portions of the first disengagement element 110 are configured to flex during operation, notably the tabs 118.

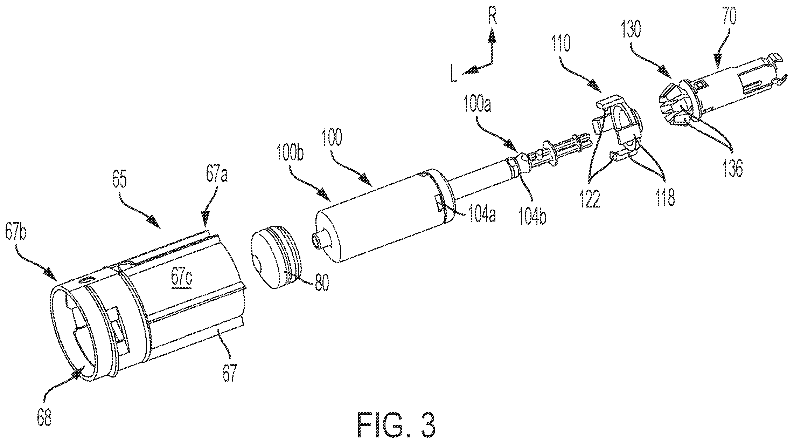

As shown in FIG. 3, the second portion 100b of the plunger actuation assembly 100 defines at least one external groove 104a extending radially inwards, where the at least one protrusion 122 of the first disengagement element 110 is configured to engage the at least one external groove 104a to translationally couple the first disengagement element 110 to the plunger actuation assembly 100. Generally, the second portion 100b of the plunger actuation assembly 100 will include a number of external grooves 104a that corresponds to the number of protrusions 122 of the first disengagement element 110. For example, in the depicted embodiment, the second portion 100b of the plunger actuation assembly 100 can define four external grooves 104a, where each protrusion 122 is configured to engage a respective one of the four external grooves 104a. As the tabs 118 and protrusions 122 can be equidistantly circumferentially spaced apart, so too can the external grooves 104a. Though it is contemplated that the external grooves can alternatively be non-equidistantly circumferentially spaced apart about the second portion 100b of the plunger actuation assembly 100, the spacing of the external grooves 104a and the tabs 118 and protrusions 122 will generally correlate. In operation, the protrusions 122 can be selectively decoupled from the corresponding external grooves 104a, so as to translationally decouple the first disengagement element 110 from the plunger actuation assembly 100.

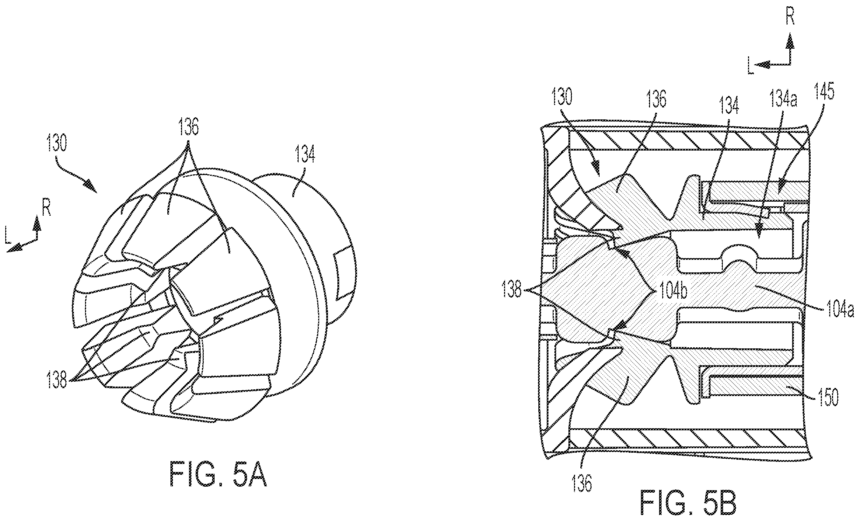

Referring to FIGS. 5A and 5B, the injection device 10 includes a second disengagement element 130. The second disengagement element 130 can define a body 134 that can be substantially disc-shaped, such that the body 134 defines a channel 134a extending therethrough. The channel 134a can be specifically sized such that the channel 134a is configured to receive a portion of the plunger actuation assembly 100. Specifically, the channel 134a can be configured to receive the first portion 100a of the plunger actuation assembly 100, such that the second disengagement element 130 is concentrically disposed around the first portion 100a of the plunger actuation assembly 100. The second disengagement element 130 can also define at least one tab 136 extending longitudinally from the body 134, and each tab 136 can include a protrusion 138 extending inwards therefrom along the radial direction R. In the depicted embodiment, the second disengagement element 110 can include eight tabs circumferentially spaced apart, and likewise eight corresponding protrusions. However, in other embodiments, it is contemplated that the second disengagement element 130 can include different numbers of tabs and protrusions, such as five, six, seven, nine, ten, eleven, etc. As depicted, the tabs 136 are equidistantly circumferentially spaced apart. In other embodiments, the tabs 136 can be non-equidistantly spaced apart circumferentially. Each component of the second disengagement element 130 can be comprised of a substantially flexible material, such as plastic, though other materials are contemplated, such that portions of second disengagement element 130 are configured to flex during operation, notably the tabs 136. The second disengagement element 130 can be coupled to the plunger actuation assembly 100 and the power pack spring 150, and thereby configured to transfer force from the power pack spring 150 to the plunger actuation assembly 100 and connected components

As shown in FIG. 5B, the first portion 100a of the plunger actuation assembly 100 defines at least one external groove 104b extending radially inwards, where the at least one protrusion 138 of the second disengagement element 130 is configured to engage the at least one external groove 104b to translationally couple the second disengagement element 130 to the plunger actuation assembly 100. The at least one external groove 104b is spaced from the at least one external groove 104a along the longitudinal direction L. Generally, the first portion 100a of the plunger actuation assembly 100 will include a single external groove 104b that extends substantially continuously circumferentially about the plunger actuation assembly 100. Alternatively, the at least one external groove 104b can include a number of external grooves 104b that corresponds to the number of tabs 136 of the second disengagement element 130. In operation, the protrusions 138 can be selectively decoupled from the corresponding external groove 104b, so as to translationally decouple the second disengagement element 130 from the plunger actuation assembly 100.

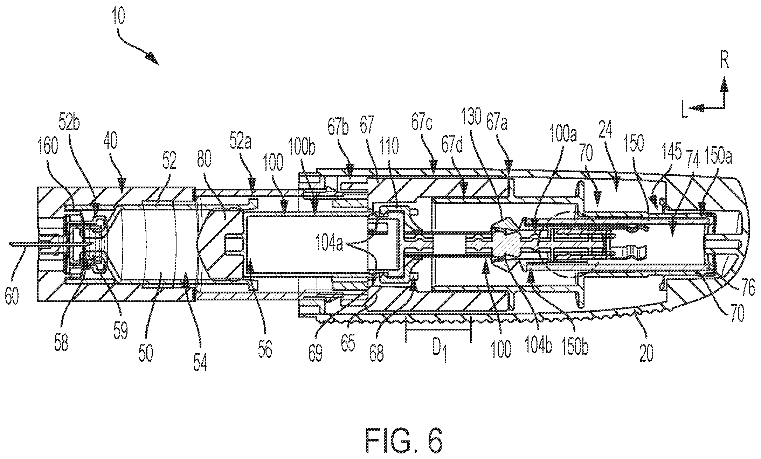

With reference to FIGS. 2 and 6-8, the operation of the injection device 10 will be described in detail. Initially, the patient can detach the cap 30 from the housing 20, thus providing access to the needle shield 40. Then, the patient can grasp the housing 20 and place the distal end of the needle shield 40 against their skin, achieving the orientation shown in FIG. 2. Next, the patient can actuate the injection device 10. To do this, the patient presses on the housing 20 of the injection device 10 in the distal direction. This decouples an activation cap 174 from a snap feature of the plunger actuation assembly 100, thus allowing retaining tabs 178 to bias inwards. By biasing inwards, the retaining tabs 178 allow expansion of the power pack spring 150.

Upon actuation of the injection device 10, the power pack spring 150 can begin to longitudinally expand. As the first end 150a of the power pack spring 150 is translationally fixed due to engagement with the ledge 76 of the proximal housing 70, the power pack spring 150 can expand through longitudinal movement of its second end 150b. As the second end 150b of the power pack spring 150 contacts a portion of the second disengagement element 130, which is (at the moment) translationally fixed to the plunger actuation assembly 100, the power pack spring 150 transfers longitudinal force to the second disengagement element 130, which likewise transfers this force to the plunger actuation assembly 100. The plunger actuation assembly 100 forces the first disengagement element 110, the cartridge 50, plunger 80, and needle 60 axially along the longitudinal direction L.

After the power pack springe 150 has driven the first and second disengagement elements 110, 130, cartridge 50, plunger 80, plunger actuation assembly 100, and needle 60 axially a first distance D₁ along the longitudinal direction L the cartridge 50 is blocked from further movement by the needle shield 40. This may occur before, after, or coincidental with the needle 60 being inserted into the skin of the patient. At this time, once the first disengagement element 110 has been driven the first distance D₁, the first disengagement element 110 is configured to engage the ledge 69 of the power pack housing 65. Engagement between the first disengagement element 110 and the ledge 69 causes the tabs 118 of the first disengagement element 110 to bias outwards. As the tabs 118 bias outwards, the protrusions 122 are moved radially out of engagement with the external grooves 104a of the plunger actuation assembly 100. As a result, the first disengagement element 110 is no longer engaged with the plunger actuation assembly 100, and is thus translationally decoupled from the plunger actuation assembly 100.

After the first disengagement element 110 is translationally decoupled from the plunger actuation assembly 100, the power pack spring 150 continues to exert force on the plunger actuation assembly 100 due to continued coupling via the second disengagement element 130. As the cartridge 50 is prevented from continued longitudinal movement, the force applied by the power pack spring 150 is transferred via the plunger actuation assembly 100 to the plunger 80, which is forced longitudinally through the chamber 54 of the cartridge 50 (which is currently in a fixed position) a second distance D₂ to force the medicament from the chamber 54, through the needle 60, and into the patient. After the plunger actuation assembly 100 is driven by the power pack spring 150 the second distance D₂, the plunger 80 reaches a final position within the cartridge 50. This final position may be the distal end of the chamber 54, as shown in FIG. 7, or may be proximal to the distal end of the chamber 54 and correspond to a predetermined maximum amount of medicament to be injected into a patient.

While the plunger actuation assembly 100 is being driven the second distance D₂, the first disengagement element 110 is in a fixed position due to its translational disengagement from the plunger actuation assembly 100. While the first disengagement element 110 is longitudinally spaced from the second disengagement element 130 a set distance as they are driven the first distance D₁, after the first and second disengagement elements 110, 130 are driven the first distance D₁, the longitudinal spacing between the first and second disengagement elements 110, 130 decreases. This is because the second disengagement element 130 remains translationally coupled to the power pack spring 150 as the plunger actuation assembly 100 is driven the second distance D₂, while the first disengagement element 110 is in a set longitudinal position, and thus the plunger actuation assembly 100 moves longitudinally relative to the first disengagement element 110. After the plunger actuation assembly 100 and the second disengagement element 130 are driven the second distance D₂, the first disengagement element 110 engages the second disengagement element 130. This engagement between the first and second disengagement elements 110, 130 is configured to bias the tabs 136 of the second disengagement element 130 outwards, such that the protrusions 138 of the second disengagement element 130 disengage the external groove 104b of the plunger actuation assembly 100, thus translationally decoupling the second disengagement element 130 from the plunger actuation assembly 100. This position is shown in FIG. 7, and corresponds to an end of the injection operation after a desired amount of the medicament has been injected.

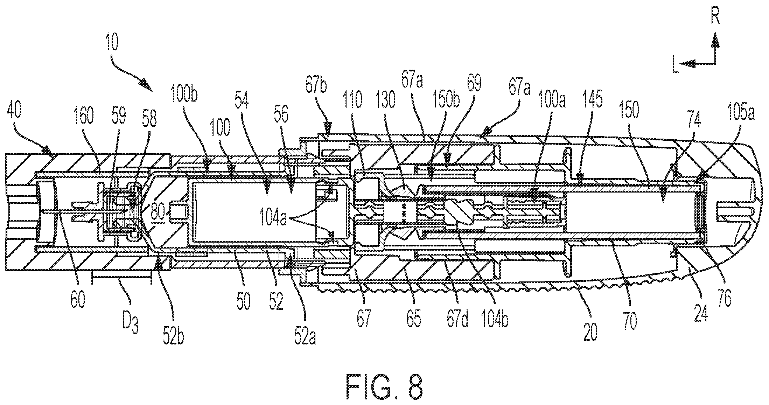

After the second disengagement element 130 translationally decouples from the plunger actuation assembly 100, the power pack spring 150 ceases to drive the plunger actuation assembly 100. This is because force from the power pack spring 150 was previously transferred to the plunger actuation assembly 100 through the second disengagement element 130. Throughout driving the above-mentioned components through the first and second distances D₁, D₂, the return spring 160 exerts a continuous force on the cartridge 50 in a direction opposed to that applied by the power pack spring 150. Despite this, the power pack spring 150 is configured to exert sufficient force to overcome the counterforce applied by the return spring 160. However, after the second disengagement element 130 is translationally decoupled from the plunger actuation assembly 100, there is no force applied to the plunger actuation assembly 100 by the power pack spring 150 to counteract the force applied by the return spring 160 on the cartridge 50. As such, as shown in FIG. 8, the return spring 160 is configured to translate the cartridge 50, plunger 80, and plunger actuation assembly 100 proximally through the housing 20 along the longitudinal direction L a third distance D₃. Simultaneously, this drives the needle 60 back within the needle shield 40 so as to protect a patient from unintended contact with the needle 60 after an injection is complete.

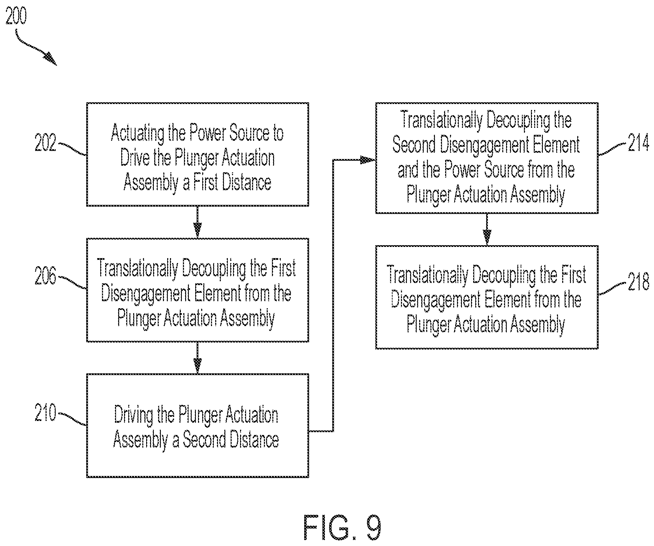

Now referring to FIG. 9, a method 200 of disconnecting the power pack spring 150 of the injection device 10 from the plunger actuation assembly 100 is shown. As stated above, the plunger actuation assembly 100 is configured to drive the plunger 80 that is disposed within the chamber 54 of the cartridge 50, where the chamber 54 is configured to hold a medicament. Method 200 begins with step 202, which involves actuating the power pack spring 150 to drive the plunger actuation assembly 100 distally a first distance D₁, where the second disengagement element 130 is configured to couple the plunger actuation assembly 100 to the power pack spring 150. Step 202 can also include driving the cartridge 50 the first distance D₁. In step 206, the first disengagement element 110 can be translationally decoupled from the plunger actuation assembly 100. Step 206 can further include engaging the first disengagement element 110 with the ledge 69 defined by the power pack housing 65, thus biasing at least one tab 118 of the first disengagement element 110 outwards and disengaging a protrusion 122 extending from the at least one tab 118 from at least one external groove 104a of the plunger actuation assembly 100. After step 202 and/or step 206, the needle 60 can be inserted a predetermined distance into the patient.

Then, step 210 includes driving the plunger actuation assembly 100 distally a second distance D₂. This step specifically involves driving the plunger 80 via the plunger actuation assembly 100 through the chamber 54 of the cartridge 50 to inject the medicament into the patient through the needle 60. During step 210, the cartridge 50 can be translationally fixed relative to the plunger actuation assembly 100. Further, during step 210, the first disengagement element 110 can be translationally fixed relative to the plunger actuation assembly 100. After step 210, in step 214 the second disengagement element 130 can be translationally decoupled, via the first disengagement element 110, from the power pack spring 150 and the plunger actuation assembly 100. Step 214 can include engaging the first and second disengagement elements 110, 130, thus biasing at least one tab 136 of the second disengagement element 130 outwards and disengaging a protrusion 138 extending from the at least one tab 136 of the second disengagement element 130 from at least one external groove 104b of the plunger actuation assembly 100. Following step 214, in step 218 the cartridge 50 and the plunger actuation assembly 100 are driven by the return spring 160 proximally a third distance D₃. After step 218, the needle 60 is retracted from the patient and into the needle shield 40, thus protecting a patient from unintended contact with the needle 60 following a completed injection.

While the invention is described herein using a limited number of embodiments, these specific embodiments are not intended to limit the scope of the invention as otherwise described and claimed herein. The precise arrangement of various elements and order of the steps of articles and methods described herein are not to be considered limiting. For instance, although the steps of the methods are described with reference to sequential series of reference signs and progression of the blocks in the figures, the method can be implemented in any particular order as desired.

## Claims

1. An injection device, comprising:
a housing (20);
a power pack housing (65) disposed within the housing (20);
a cartridge (50) defining a chamber (54) configured to hold a medicament;
a plunger (80) disposed within the chamber (54);
a plunger actuation assembly (100) configured to drive the plunger (80);
a power source (145) configured to drive the plunger actuation assembly (100) such that the plunger (80) forces the medicament from the chamber (54);
a first disengagement element (110) translationally fixed relative to the plunger actuation assembly (100); and
a second disengagement element (130) coupled to the plunger actuation assembly (100) and to the power source (145), wherein the second disengagement element (130) is translationally fixed relative to the plunger actuation assembly (100),
wherein the first disengagement element (110) is configured to:
translationally decouple from the plunger actuation assembly (100) by an engagement with the power pack housing (65) when the power source (145) drives the plunger actuation assembly (100) a first distance, and
translationally decouple the second disengagement element (130) from the plunger actuation assembly (100) by an engagement with the second disengagement element (130) when the power source (145) drives the plunger actuation assembly (100) a second distance.

2. The injection device of claim 1, wherein the power source (145) is a spring (150).

3. The injection device of claim 2, wherein the plunger actuation assembly (100) has a body defining at least one first external groove (104a),
wherein the first disengagement element (110) includes a body (114) defining a channel (114a) extending therethrough, the channel (114a) being configured to receive the plunger actuation assembly (100), at least one tab (118) extending longitudinally from the body (114), and a projection (122) extending radially inward from the at least one tab (118) and configured to engage the at least one groove (104a) to translationally couple the first disengagement element (110) to the plunger actuation assembly (100).

4. The injection device of claim 3, wherein the at least one tab (118) includes four tabs (118) circumferentially spaced apart and the at least one first external groove (104a) includes four external grooves (104a), wherein a respective projection (122) extends radially from each of the four tabs (118); and optionally,
wherein the four tabs (118) are equidistantly circumferentially spaced apart.

5. The injection device of claim 3, wherein the power pack housing (65) has a body (67) defining a first end (67a), a second end (67b) opposite the first end (67a), and an inner surface (67d) defining a channel (68) extending through the body (67) from the first end (67a) to the second end (67b) and a ledge (69) facing the channel (68).

6. The injection device of claim 5, wherein the ledge (69) extends circumferentially about the entirety of the inner surface (67d).

7. The injection device of claim 5, wherein the first disengagement element (110) is configured to engage the ledge (69) when the spring (150) drives the plunger actuation assembly (100) the first distance, wherein engagement between the first disengagement element (110) and the ledge (69) biases the at least one tab (118) outwards such that the projection (122) disengages the at least one first external groove (104a) of the plunger actuation assembly (100), thus translationally decoupling the first disengagement element (110) from the plunger actuation assembly (100).

8. The injection device of claim 7, wherein the body of the plunger actuation assembly (100) defines at least one second external groove (104b) longitudinally spaced from the at least one first external groove (104a),
wherein the second disengagement element (130) includes a body (134) defining a channel (134a) extending therethrough, the channel (134a) being configured to receive the plunger actuation assembly (100), at least one tab (136) extending longitudinally from the body (134), and a projection (138) extending radially inward from the at least one tab (136) and configured to engage the at least one second external groove (104b) to translationally couple the second disengagement element (130) to the plunger actuation assembly (100).

9. The injection device of claim 8, wherein the first disengagement element (110) is configured to engage the second disengagement element (130) when the spring (150) drives the plunger actuation assembly (100) the second distance, wherein engagement between the first and second disengagement elements (110, 130) is configured to bias the at least one tab (136) of the second disengagement element (130) outwards such that the projection disengages the at least one second groove of the plunger actuation assembly (100), thus translationally decoupling the second disengagement element (130) from the plunger actuation assembly (100).

10. The injection device of claim 9, wherein the at least one tab (136) of the second disengagement element (130 includes eight tabs (136) radially spaced apart and the at least one second external groove (104b) extends substantially continuously circumferentially about the plunger actuation assembly (100).

11. The injection device of claim 10, wherein the eight tabs (136) of the second disengagement element (130) are equidistantly circumferentially spaced apart.

12. The injection device of claim 2, wherein the spring (150) ceases to drive the plunger actuation assembly (100) after the second disengagement element (130) is translationally decoupled from the plunger actuation assembly (100).

13. The injection device of claim 2, further comprising:
a needle (60) extending from the cartridge (50),
wherein the spring (150) is configured to drive the plunger actuation assembly (100) and the cartridge (50) the first distance to insert the needle (60) into a user, and the spring (150) is configured to drive the plunger actuation assembly (100) and the plunger the second distance to inject the medicament into the user.

14. The injection device of claim 1, wherein the first disengagement element (110) is longitudinally spaced from the second disengagement element (130) as it moves the first distance.

15. The injection device of claim 2, wherein the first disengagement element (110) is translationally decoupled from the plunger actuation assembly (100) as the spring (150) drives the plunger actuation assembly (100) the second distance.

## Patentansprüche

1. Injektionsvorrichtung, umfassend:
ein Gehäuse (20);
ein Powerpackgehäuse (65), das in dem Gehäuse (20) angeordnet ist;
eine Kartusche (50), die eine Kammer (54) definiert, die dazu ausgelegt ist, ein Medikament zu enthalten;
einen Kolben (80), der in der Kammer (54) angeordnet ist;
eine Kolbenbetätigungsanordnung (100), die dazu ausgelegt ist, den Kolben (80) anzutreiben;
eine Energiequelle (145), die dazu ausgelegt ist, die Kolbenbetätigungsanordnung (100) derart anzutreiben, dass der Kolben (80) das Medikament aus der Kammer (54) heraus treibt;
ein erstes Entkopplungselement (110), das bezogen auf die Kolbenbetätigungsanordnung (100) translatorisch fixiert ist; und
ein zweites Entkopplungselement (130), das mit der Kolbenbetätigungsanordnung (100) und mit der Energiequelle (145) gekoppelt ist, wobei das zweite Entkopplungselement (130) bezogen auf die Kolbenbetätigungsanordnung (100) translatorisch fixiert ist,
wobei das erste Entkopplungselement (110) dazu ausgelegt ist:
sich durch einen Eingriff mit dem Powerpackgehäuse (65) translatorisch von der Kolbenbetätigungsanordnung (100) zu trennen, wenn die Energiequelle (145) die Kolbenbetätigungsanordnung (100) eine erste Distanz antreibt, und
das zweite Entkopplungselement (130) durch einen Eingriff mit dem zweiten Entkopplungselement (130) translatorisch von der Kolbenbetätigungsanordnung (100) zu trennen, wenn die Energiequelle (145) die Kolbenbetätigungsanordnung (100) eine zweite Distanz antreibt.

2. Injektionsvorrichtung nach Anspruch 1, wobei die Energiequelle (145) eine Feder (150) ist.

3. Injektionsvorrichtung nach Anspruch 2, wobei die Kolbenbetätigungsanordnung (100) einen Körper hat, der mindestens eine erste externe Nut (104a) definiert,
wobei das erste Entkopplungselement (110) einen Körper (114) aufweist, der einen Kanal (114a) definiert, der sich dort hindurch erstreckt, wobei der Kanal (114a) dazu ausgelegt ist, die Kolbenbetätigungsanordnung (100) aufzunehmen, mindestens eine Zunge (118), die sich in Längsrichtung von dem Körper (114) erstreckt, und einen Vorsprung (122), der sich von der mindestens einen Zunge (118) radial einwärts erstreckt und dazu ausgelegt ist, die mindestens eine Nut (104a) in Eingriff zu nehmen, um das erste Entkopplungselement (110) mit der Kolbenbetätigungsanordnung (100) zu koppeln.

4. Injektionsvorrichtung nach Anspruch 3, wobei die mindestens eine Zunge (118) vier Zungen (118) aufweist, die in Umfangsrichtung beabstandet sind, und die mindestens eine erste externe Nut (104a) vier externe Nuten (104a) aufweist, wobei sich ein entsprechender Vorsprung (122) von jeder der vier Zungen (118) radial erstreckt; und optional
wobei die vier Zungen (118) in Umfangsrichtung gleichmäßig voneinander beabstandet sind.

5. Injektionsvorrichtung nach Anspruch 3, wobei das Powerpackgehäuse (65) einen Körper (67) hat, der ein erstes Ende (67a), ein zweites Ende (67b) gegenüber dem ersten Ende (67a) und eine Innenfläche (67d), die einen Kanal (68) definiert, der sich von dem ersten Ende (67a) zu dem zweiten Ende (67b) durch den Körper (67) erstreckt, und eine Kante (69), die dem Kanal (68) zugewandt ist.

6. Injektionsvorrichtung nach Anspruch 5, wobei sich die Kante (69) in Umfangsrichtung um die gesamte Innenfläche (67d) erstreckt.

7. Injektionsvorrichtung nach Anspruch 5, wobei das erste Entkopplungselement (110) dazu ausgelegt ist, die Kante (69) in Eingriff zu nehmen, wenn die Feder (150) die Kolbenbetätigungsanordnung (100) die erste Distanz antreibt, wobei der Eingriff zwischen dem ersten Entkopplungselement (110) und der Kante (69) die mindestens eine Zunge (118) derart nach außen vorspannt, dass der Vorsprung (122) die mindestens eine externe Nut (104a) der Kolbenbetätigungsanordnung (100) entkoppelt und somit das erste Entkopplungselement (110) von der Kolbenbetätigungsanordnung (100) translatorisch trennt.

8. Injektionsvorrichtung nach Anspruch 7, wobei der Körper der Kolbenbetätigungsanordnung (100) mindestens eine zweite externe Nut (104b) definiert, die in Längsrichtung von der mindestens einen ersten externen Nut (104a) beabstandet ist,
wobei das zweite Entkopplungselement (130) einen Körper (134) aufweist, der einen Kanal (134a) definiert, der sich dort hindurch erstreckt, wobei der Kanal (134a) dazu ausgelegt ist, die Kolbenbetätigungsanordnung (100) aufzunehmen, mindestens eine Zunge (136), die sich in Längsrichtung von dem Körper (134) erstreckt, und einen Vorsprung (138), der sich von der mindestens einen Zunge (136) radial nach innen erstreckt und dazu ausgelegt ist, die mindestens eine zweite externe Nut (104b) in Eingriff zu nehmen, um das zweite Entkopplungselement (130) mit der Kolbenbetätigungsanordnung (100) translatorisch zu koppeln.

9. Injektionsvorrichtung nach Anspruch 8, wobei das erste Entkopplungselement (110) dazu ausgelegt ist, das zweite Entkopplungselement (130) in Eingriff zu nehmen, wenn die Feder (150) die Kolbenbetätigungsanordnung (100) die zweite Distanz antreibt, wobei der Eingriff zwischen dem ersten und dem zweiten Entkopplungselement (110, 130) dazu ausgelegt ist, die mindestens eine Zunge (136) des zweiten Entkopplungselements (130) derart nach außen vorzuspannen, dass der Vorsprung die mindestens eine zweite Nut der Kolbenbetätigungsanordnung (100) entkoppelt und somit das zweite Entkopplungselement (130) von der Kolbenbetätigungsanordnung (100) trennt.

10. Injektionsvorrichtung nach Anspruch 9, wobei die mindestens eine Zunge (136) des zweiten Entkopplungselements (130) acht Zungen (136) aufweist, die radial beabstandet sind und sich die mindestens eine zweite externe Nut (104b) im Wesentlichen in Umfangsrichtung um die Kolbenbetätigungsanordnung (100) erstreckt.

11. Injektionsvorrichtung nach Anspruch 10, wobei die acht Zungen (136) des zweiten Entkopplungselements (130) in Umfangsrichtung gleichmäßig voneinander beabstandet sind.

12. Injektionsvorrichtung nach Anspruch 2, wobei die Feder (150) aufhört, die Kolbenbetätigungsanordnung (100) anzutreiben, nachdem das zweite Entkopplungselement (130) translatorisch von der Kolbenbetätigungsanordnung (100) getrennt ist.

13. Injektionsvorrichtung nach Anspruch 2, ferner umfassend:
eine Nadel (60), die sich von der Kartusche (50) erstreckt,
wobei die Feder (150) dazu ausgelegt ist, die Kolbenbetätigungsanordnung (100) und die Kartusche (50) die erste Distanz anzutreiben, um die Nadel (60) in einen Benutzer einzuführen, und die Feder (150) dazu ausgelegt ist, die Kolbenbetätigungsanordnung (100) und den Kolben die zweite Distanz anzutreiben, um das Medikament in den Benutzer zu injizieren.

14. Injektionsvorrichtung nach Anspruch 1, wobei das erste Entkopplungselement (110) in Längsrichtung von dem zweiten Entkopplungselement (130) beabstandet ist, wenn es sich die erste Distanz bewegt.

15. Injektionsvorrichtung nach Anspruch 2, wobei das erste Entkopplungselement (110) translatorisch von der Kolbenbetätigungsanordnung (100) getrennt wird, wenn die Feder (150) die Kolbenbetätigungsanordnung (100) die zweite Distanz antreibt.

## Revendications

1. Dispositif d'injection, comprenant :
un boîtier (20) ;
un boîtier de bloc d'alimentation (65) disposé à l'intérieur du boîtier (20) ;
une cartouche (50) définissant une chambre (54) configurée pour contenir un médicament ;
un piston (80) disposé à l'intérieur de la chambre (54) ;
un ensemble d'actionnement de piston (100) configuré pour entraîner le piston (80) ;
une source de puissance (145) configurée pour entraîner l'ensemble d'actionnement de piston (100) de telle sorte que le piston (80) pousse le médicament depuis la chambre (54) ;
un premier élément de désaccouplement (110) fixe en translation par rapport à l'ensemble d'actionnement de piston (100) ; et
un second élément de désaccouplement (130) couplé à l'ensemble d'actionnement de piston (100) et à la source de puissance (145), le second élément de désaccouplement (130) étant fixe en translation par rapport à l'ensemble d'actionnement de piston (100),
le premier élément de désaccouplement (110) étant configuré pour :
se désaccoupler en translation de l'ensemble d'actionnement de piston (100) par une mise en prise avec le boîtier de bloc d'alimentation (65) lorsque la source de puissance (145) entraîne l'ensemble d'actionnement de piston (100) à une première distance, et
désaccoupler en translation le second élément de désaccouplement (130) de l'ensemble d'actionnement de piston (100) par une mise en prise avec le second élément de désaccouplement (130) lorsque la source de puissance (145) entraîne l'ensemble d'actionnement de piston (100) à une seconde distance.

2. Dispositif d'injection selon la revendication 1, la source de puissance (145) étant un ressort (150).

3. Dispositif d'injection selon la revendication 2, l'ensemble d'actionnement de piston (100) ayant un corps définissant au moins une première rainure externe (104a), le premier élément de désaccouplement (110) comprenant un corps (114) définissant un canal (114a) s'étendant à travers celui-ci, le canal (114a) étant configuré pour recevoir l'ensemble d'actionnement de piston (100), au moins une languette (118) s'étendant longitudinalement depuis le corps (114), et une saillie (122) s'étendant radialement vers l'intérieur depuis l'au moins une languette (118) et configurée pour venir en prise avec l'au moins une rainure (104a) pour coupler en translation le premier élément de désaccouplement (110) à l'ensemble d'actionnement de piston (100).

4. Dispositif d'injection selon la revendication 3, l'au moins une languette (118) comprenant quatre languettes (118) espacées circonférentiellement et l'au moins une première rainure externe (104a) comprenant quatre rainures externes (104a), une saillie respective (122) s'étendant radialement depuis chacun des quatre languettes (118) ; et éventuellement,
les quatre languettes (118) étant espacées circonférentiellement de manière équidistante.

5. Dispositif d'injection selon la revendication 3, le boîtier de bloc d'alimentation (65) ayant un corps (67) définissant une première extrémité (67a), une seconde extrémité (67b) opposée à la première extrémité (67a), et une surface interne (67d) définissant un canal (68) s'étendant à travers le corps (67) de la première extrémité (67a) à la seconde extrémité (67b) et un rebord (69) faisant face au canal (68).

6. Dispositif d'injection selon la revendication 5, le rebord (69) s'étendant circonférentiellement autour de la totalité de la surface interne (67d).

7. Dispositif d'injection selon la revendication 5, le premier élément de désaccouplement (110) étant configuré pour venir en prise avec le rebord (69) lorsque le ressort (150) entraîne l'ensemble d'actionnement de piston (100) à la première distance, la mise en prise entre le premier élément de désaccouplement (110) et le rebord (69) sollicitant l'au moins une languette (118) vers l'extérieur de telle sorte que la saillie (122) se désengage de l'au moins une première rainure externe (104a) de l'ensemble d'actionnement de piston (100), désaccouplant en translation ainsi le premier élément de désaccouplement (110) de l'ensemble d'actionnement de piston (100).

8. Dispositif d'injection selon la revendication 7, le corps de l'ensemble d'actionnement de piston (100) définissant au moins une seconde rainure externe (104b) espacée longitudinalement de l'au moins une première rainure externe (104a),
le second élément de désaccouplement (130) comprenant un corps (134) définissant un canal (134a) s'étendant à travers celui-ci, le canal (134a) étant configuré pour recevoir l'ensemble d'actionnement de piston (100), au moins une languette (136) s'étendant longitudinalement depuis le corps (134), et une saillie (138) s'étendant radialement vers l'intérieur depuis l'au moins une languette (136) et configurée pour venir en prise avec l'au moins une seconde rainure externe (104b) pour coupler en translation le second élément de désaccouplement (130) à l'ensemble d'actionnement de piston (100).

9. Dispositif d'injection selon la revendication 8, le premier élément de désaccouplement (110) étant configuré pour venir en prise avec le second élément de désaccouplement (130) lorsque le ressort (150) entraîne l'ensemble d'actionnement de piston (100) à la seconde distance, la mise en prise entre les premier et second éléments de désaccouplement (110, 130) étant configuré pour solliciter l'au moins une languette (136) du second élément de désaccouplement (130) vers l'extérieur de telle sorte que la saillie se désengage de l'au moins une seconde rainure de l'ensemble d'actionnement de piston (100), désaccouplant en translation ainsi le second élément de désaccouplement (130) de l'ensemble d'actionnement de piston (100).

10. Dispositif d'injection selon la revendication 9, l'au moins une languette (136) du second élément de désaccouplement (130) comprenant huit languettes (136) espacées radialement et l'au moins une seconde rainure externe (104b) s'étendant sensiblement de manière continue circonférentiellement autour de l'ensemble d'actionnement de piston (100).

11. Dispositif d'injection selon la revendication 10, les huit languettes (136) du second élément de désaccouplement (130) étant espacées circonférentiellement de manière équidistante.

12. Dispositif d'injection selon la revendication 2, le ressort (150) cessant d'entraîner l'ensemble d'actionnement de piston (100) après que le second élément de désaccouplement (130) soit désaccouplé en translation de l'ensemble d'actionnement de piston (100).

13. Dispositif d'injection selon la revendication 2, comprenant en outre :
une aiguille (60) s'étendant depuis la cartouche (50),
le ressort (150) étant configuré pour entraîner l'ensemble d'actionnement de piston (100) et la cartouche (50) à la première distance pour insérer l'aiguille (60) dans un utilisateur, et le ressort (150) étant configuré pour entraîner l'ensemble d'actionnement de piston (100) et le piston à la seconde distance pour injecter le médicament dans l'utilisateur.

14. Dispositif d'injection selon la revendication 1, le premier élément de désaccouplement (110) étant espacé longitudinalement du second élément de désaccouplement (130) lorsqu'il se déplace à la première distance.

15. Dispositif d'injection selon la revendication 2, le premier élément de désaccouplement (110) étant désaccouplé en translation de l'ensemble d'actionnement de piston (100) lorsque le ressort (150) entraîne l'ensemble d'actionnement de piston (100) à la seconde distance.
